# FASCICULE DE BREVET EUROPEEN

(11) **EP 3 003 399 B1**
(45) Date de publication et mention de la délivrance du brevet: **01.02.2017**
(21) Numéro de dépôt: 14732508.8
(22) Date de dépôt: 27.05.2014
(51) Int. Cl.: A61K 51/04, C07C 211/38, A61K 101/02

(54) **NOUVEAUX COMPOSES CHIMIQUES DERIVES DE LA NORMEMANTINE ET LEUR UTILISATION DANS LE DOMAINE MEDICAL**
NEUARTIGE CHEMISCHE VERBINDUNGEN AUS NORMEMANTIN UND VERWENDUNG DAVON IM MEDIZINISCHEN BEREICH
NOVEL CHEMICAL COMPOUNDS DERIVED FROM NORMEMANTINE AND USE OF SAME IN THE MEDICAL FIELD

(30) Priorité: 29.05.2013 FR 1301216
(43) Date de publication de la demande: 13.04.2016
(73) Titulaire: M2I DEVELOPMENT, 64170 Lacq (FR)
(72) Inventeur: GUERRET, Olivier, F-46170 Pern (FR); DUFOUR, Samuel, F-64300 Orthez (FR); BELHADJ-TAHAR, Hafid, F-31100 Toulouse (FR)
(74) Mandataire: Regimbeau
(86) Numéro de dépôt international: PCT/EP2014/060981
(87) Numéro de publication internationale: WO 2014/191424

(56) Documents cités:
- AMETAMEY S M ET AL: "PET studies of <18>F-memantine in healthy volunteers", NUCLEAR MEDICINE AND BIOLOGY, ELSEVIER, NY, US, vol. 29, no. 2, 1 février 2002 (2002-02-01), pages 227-231, XP004334821, ISSN: 0969-8051, DOI: 10.1016/S0969-8051(01)00293-1 cité dans la demande
- AMETAMEY S M ET AL: "Fluorine-18 radiolabelling, biodistribution studies and preliminary PET evaluation of a new memantine derivative for imaging the NMDA receptor.", JOURNAL OF RECEPTOR AND SIGNAL TRANSDUCTION RESEARCH 1999 JAN-JUL, vol. 19, no. 1-4, janvier 1999 (1999-01), pages 129-141, XP008167695, ISSN: 1079-9893
- SAMNICK, SAMUEL ET AL: "Electrophysiological study, biodistribution in mice, and preliminary PET evaluation in a rhesus monkey of 1-amino-3-[18F]fluoromethyl-5-methyl- adamantane (18F-MEM): a potential radioligand for mapping the NMDA-receptor complex", NUCLEAR MEDICINE AND BIOLOGY , 25(4), 323-330 CODEN: NMBIEO; ISSN: 0969-8051, 1998, XP002720893, DOI: 10.1016/S0969-8051(98)00003-1 cité dans la demande
- SAMNICK, SAMUEL ET AL: "Synthesis and preliminary in vitro evaluation of a new memantine derivative 1-amino-3-[18F]fluoromethyl-5-methyladaman tane: a potential ligand for mapping the N-methyl-D-aspartate receptor complex", JOURNAL OF LABELLED COMPOUNDS & RADIOPHARMACEUTICALS , 39(3), 241-250 CODEN: JLCRD4; ISSN: 0362-4803, 1997, XP002720894, DOI: 10.1002/(SICI)1099-1344(199703)39:3<241::A ID-JLCR966>3.0.CO;2-V cité dans la demande
- ZDANYS K ET AL: "A systematic review of off-label uses of memantine for psychiatric disorders", PROGRESS IN NEURO-PSYCHOPHARMACOLOGY AND BIOLOGICAL PSYCHIATRY 20080801 US, vol. 32, no. 6, 1 août 2008 (2008-08-01), pages 1362-1374, XP023181310, ISSN: 0278-5846 cité dans la demande

## Description

La présente invention concerne de nouveaux composés chimiques dérivés de la mémantine et susceptibles de se fixer sur les récepteurs de N-Méthyl-D-Aspartate (NMDA), qui sont activés par leur liaison avec le neurotransmetteur glutamate. Une hyper activation des récepteurs NMDA par le glutamate est directement corrélée à des maladies neurologiques.

De par l'affinité des dérivés de la mémantine pour les récepteurs NMDA, l'invention concerne également des marqueurs de ces récepteurs utilisables pour de l'imagerie médicale. Les précurseurs de ces dérivés sont aussi objets de l'invention.

Il est connu que les récepteurs NMDA sont impliqués dans les phénomènes d'apprentissage et de mémoire (Nature, 319 (1986), p. 774-776). Les récepteurs NMDA sont des canaux dont l'ouverture anormale entraine des désordres neuro-dégénératifs, par exemple les maladies de Parkinson, d'Alzheimer ou de Huntington. Ils sont aussi impliqués dans des désordres neurologiques tels que l'épilepsie, l'hypoglycémie, l'ischémie périnatale, ou des troubles liés à des traumatismes crâniens, de la moelle épinière, ou à des crises cardiaques. On a donc recherché l'action d'antagonistes des récepteurs NMDA, mais ces antagonistes avaient souvent des effets secondaires indésirables, qui empêchaient de prendre en considération leur éventuelle utilisation dans un traitement thérapeutique. On a constaté que la mémantine est un antagoniste non compétitif des récepteurs NMDA et que ses effets secondaires, aux doses thérapeutiques envisagées, étaient plus réduits que ceux des antagonistes antérieurement connus (Neuropharmacologie, Vol. 38(6), 15 Juin 1999, p. 735-767).

Les récepteurs NMDA, contrairement aux autres récepteurs ionotropiques du glutamate, requièrent, pour leur activation, une liaison simultanée du glutamate et d'un co antagoniste, la glycine : on a donc cherché à mettre en évidence un certain nombre de produits bloquant les canaux NMDA par un marquage au carbone 11 ou au fluor 18 ou à l'iode 123 : les résultats initiaux n'étaient pas satisfaisants parce que les radio ligands n'étaient pas spécifiques ou parce que le radio élément choisi avait une durée de vie trop courte pour permettre un protocole « fabrication-injection-détection » suffisamment court pour une détection efficace.

Parmi tous les résultats, on a obtenu les meilleurs en utilisant la ¹⁸F-mémantine, qui est un antagoniste ayant pour le récepteur NMDA une affinité similaire à celle de la mémantine elle-même (voir par exemple S. Samnick, S. Ametamey, M. Gold, P. Schubiger Journal of Labelled compounds and radiopharmaceuticals, vol. 29, n°3, p. 241-250, Samnick S., Ametamey S., Gold M.R., Schubiger P.A. J. Lab. Comp. Radiopharm., 39, 241-250 (1997) ou des mêmes auteurs Nucl Med Biol. 1998 May;25(4):323-30). La détection du bloqueur marqué quand il est lié au récepteur a été faite avec un scanner à émission de positrons (« scanner PET ») (Nuclear Medecine and Biology 29 (2002), p 227-231). Ametamey et al (J. of receptor and signal transduction research, vol. 19, No. 1-4, pages 129-141, 1999) ont décrit la préparation de la ¹⁸F-mémantine et présentent des études de biodistribution et d'imagerie PET chez le singe Rhésus.

Il s'est donc avéré que l'utilisation de la ¹⁸F-mémantine pouvait être envisagée dans l'étude de diverses maladies neurologiques, mais il reste néanmoins un certain nombre d'effets secondaires (notamment céphalées, vertiges, somnolence, hallucinations), qui empêchent de recommander la mémantine de façon générale (Progress in Neuro-psychopharmacology and Biological Psychiatry, 32 (2008), p. 1362-1374). En fait, la mémantine a des effets secondaires plus faibles que les bloqueurs connus des canaux de récepteurs NMDA, telle que la kétamine, en raison du fait que la mécanique de blocage n'est pas la même : la kétamine reste bloquée dans les récepteurs NMDA quand l'antagoniste disparaît alors que, pour la mémantine, il n'y a qu'un piégeage résiduel partiel (J. Physiol. 587, 19(2009), p. 4489-4603).

Il apparaît donc que les différents antagonistes des récepteurs NMDA n'agissent pas d'une seule façon, de sorte que les intérêts et les désavantages d'un antagoniste ne se retrouvent pas nécessairement pour un autre antagoniste, et ce même si les structures sont proches.

Par exemple, dans le but de maximiser le radio marquage des récepteurs NMDA, il est important que le marqueur, une fois injecté, se concentre dans le cerveau plutôt que dans d'autres organes (poumon, rein, foie). A titre d'exemple la ¹⁸F fluoro mémantine a une distribution de 3.6% ID/g dans le cerveau 60mn après injection avec un rapport Cerveau/sang qui augmente dans le temps : 2.40, 5.10, 6.33, et 9.27 pour 5, 30, 60, et 120 mn. Ce rendement est assez faible et trouver des dérivés permettant une concentration dans le cerveau plus élevée reste un enjeu pour l'étude des récepteurs NMDA.

Dans le but de maximiser l'efficacité de traçage des récepteurs NMDA, il est donc important de trouver de nouvelles molécules marquées caractérisées en ce qu'elles sont accessibles avec de bons rendement dans un délai compatible avec la durée de vie du ¹⁸F, et présentant à la fois une bonne affinité pour les récepteurs NMDA et une affinité pour le cerveau suffisante par rapport au sang et aux autres organes.

Selon l'invention, la demanderesse a découvert un nouveau dérivé de la mémantine comportant un atome de ¹⁸F qui est facilement accessible à partir d'un précurseur et présentant une bonne affinité pour le cerveau et une bonne affinité pour les récepteurs NMDA-différentes de celles des composés de la littérature cités précédemment.

Ces différences peuvent être exploitées pour le marquage des récepteurs NMDA et leur visualisation via des PET scan pour étudier ces récepteurs et leur réaction à des traitements médicamenteux ou dans le développement de maladies neurodégénératives.

La présente invention a donc pour premier objet un composé chimique nouveau correspondant à la formule (I), aussi nommée 2-fluoroethyl Normémantine : 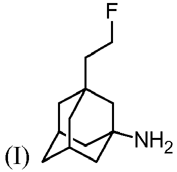 La molécule (I) peut être en équilibre avec une forme protonée de formule (II) : 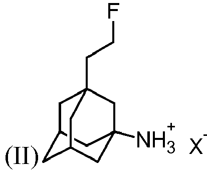 formule dans laquelle, X⁻ désigne un contre anion issu du milieu biologique ou choisi parmi les ions chlorure, bromure, iodure, acétate, méthane sulfonate, benzène sulfonate, camphosulphonate, tartrate, dibenzoate, ascorbate, fumarate, citrate, phosphate, salicylate, oxalate, bromohydrate, tosylate.Le produit de formule (II) est donc un sel du produit de formule (I).

L'invention concerne également les deux précurseurs des molécules (I) et (II). Le premier composé est le composé (P1) de formule : 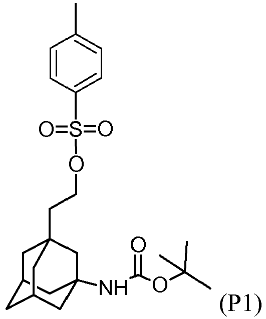 Le second composé est le composé (P2) de formule :

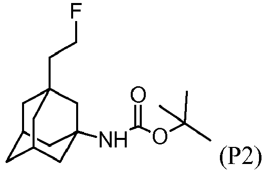

L'invention concerne également une utilisation du composé (I) ou (II), dans lequel l'atome de fluor indiqué dans la formule est de préférence un atome de ¹⁸F, caractérisée en ce que l'on injecte une solution aqueuse de l'un de ces dérivés ou de leur mélange dans le système circulatoire sanguin d'un sujet vivant pour permettre la détection de ses récepteurs NMDA, au moyen d'un scanner à émission de positron, par repérage des atomes de ¹⁸F du composé (I) ou (II) et d'étudier leur comportement dans des études sur des maladies psychiatriques.

Dans un mode de réalisation particulier, la présente invention concerne une solution aqueuse du composé (I) ou du composé (II), en particulier une solution aqueuse pour être injectée en intraveineuse. L'invention vise aussi l'utilisation des composés (I), (II), (P1) ou (P2) dans des compositions médicamenteuses destinées au traitement de maladies neurologiques

La présente invention vise aussi l'utilisation de solution aqueuse du composé (I) ou du composé (II) en injection à un mammifère pour réaliser un scanner à émission de positron, par repérage des atomes de ¹⁸F pour étudier le comportement des récepteurs NMDA lors d'étude sur des maladies neurologiques.

L'invention concerne également le composé (I) ou le composé (II) pour son utilisation en tant que médicament.

### Exemple 1: Procédé de préparation du 3-Amino-1-adamantane éthanol (B)

On utilise comme produit de départ le chlorhydrate de formule (A): 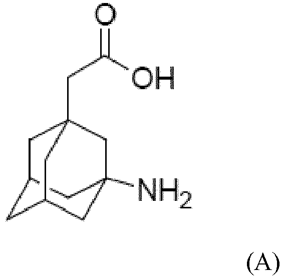 qui est un produit commercialisé par la société Alinda sous la référence HLS0044-025.

Dans un ballon tricol de 50 ml, on suspend 338 mg de produit (A) (1,38mmol) dans 22 ml de tétrahydrofurane anhydre, sous atmosphère d'argon. Le ballon est équipé d'un condenseur et d'un thermomètre ; la suspension est refroidie à 0°C. On ajoute alors goutte à goutte, 0,66 ml de BH₃SMe₂ (6,9 mmol) : la réaction est très exothermique et on refroidit le réacteur de manière à maintenir le milieu réactionnel en dessous de 5°C. L'addition est réalisée en 30 mn et l'agitation à basse température est maintenue pendant 8h puis on laisse la température revenir à température ambiante pendant 8 heures. On chauffe ensuite pour maintenir le milieu réactionnel à 50°C pendant 16 heures pour achever la réaction de fixation des molécules (A) sur le borane-diméthyl-sulfure.

On refroidit ensuite à 0°C et on hydrolyse le complexe au moyen d'une introduction lente de 4,5 ml de méthanol. On ajoute ensuite au milieu réactionnel une solution aqueuse de soude à 10% de façon à obtenir un pH supérieur à 10. On maintient sous agitation pendant 1 heure puis on concentre à sec à 40°C sous pression réduite de 1 kPa.

Le résidu est alors repris dans 6 ml d'eau qui est ensuite acidifiée au moyen d'une solution acide chlorhydrique 33% jusqu'à obtenir un pH de 1. La phase aqueuse est lavée trois fois avec 6 ml de methyl-tertiobutyl-ether. La solution aqueuse est alors portée à un pH de 10 au moyen d'une solution 30% de soude. Le produit (B) est ensuite récupéré par 3 extractions avec 6 ml de dichlorométhane. Les fractions sont rassemblées et évaporées sous pression réduite de 1 kPa jusqu'à obtention d'un solide blanc (127 mg, rendement : 47%).

Le produit est caractérisé par RMN :
¹H RMN (CDC13, Bruker 400 MHz) 3,72 (2H, dt J=12Hz, CH₂OH), 2,8 (1H, OH), 2,21 (2H, large, NH2), 0,75-1,40 (14H, CH₂).

### Exemple 2 : Préparation N(3-ethanol adamantane)-O-tertiobutyl carbamate (C)

Le produit (B) obtenu dans l'exemple 1 est utilisé comme produit de départ.

195 mg de composé (B) sont dilués dans 1 ml de dioxane et 1 ml d'eau. On ajoute ensuite 260 mg carbonate de soude puis 400 mg de di-ter-butyl- dicarbonate en solution dans 2 ml de dioxane. Le milieu est maintenu sous agitation pendant 72 heures.

On ajoute ensuite 10 ml d'eau au milieu dont on ajuste le pH à 3 au moyen d'une solution d'acide chlorhydrique 1 N. Le produit est ensuite extrait par des extractions successives avec 10 ml d'acétate d'éthyle. Les extraction sont rassemblées, séchées sur MgSO4 puis le produit (C) est récupéré par évaporation sous pression réduite à 1 kPa.

Le produit (C) est obtenu sous forme d'un solide blanc (250 mg, rendement 84%) et est caractérisé par RMN:
¹H RMN (CDCl3, Bruker 300 MHz) 4,35 (1H, br, NH) ; 3,7 (2H, dt J=7 Hz, CH₂OH), 2,65 (1H, OH), 1,2-1,85 (29H, CH₂).

### Exemple 3 : Préparation N(3-(2-tosylate-ethyl) adamantane)-O-tertiobutyl carbamate (P1)

Le produit (C) obtenu dans l'exemple 2 est utilisé comme produit de départ.

295 mg de composé C sont dilués dans 1 ml de dichlorométhane. On ajoute ensuite 200 mg de triéthyl amine dilués dans 1 ml de dichlorométhane. On ajoute ensuite 200 mg de chlorure de tosyle dilués dans 2 ml de dichlorométhane. Le milieu est maintenu sous agitation pendant 24 heures.

On lave le milieu avec 3 fois 5 ml d'eau à pH acide puis 3 fois avec 5ml à pH 7. La phase organique est séchée sur MgSO₄ puis le produit P1 est récupéré par évaporation sous pression réduite à 1 kPa.

Le produit (P1) est obtenu sous forme d'un solide blanc (425 mg, rendement 95%) et est caractérisé par RMN:
¹H RMN (CDCl3, Bruker 300 MHz) : 7,79 (2H, d, J=8,1 Hz, CH aromatique), 7,36 (2H, d, J=8,1 Hz, 2 CH aromatique), 4,34 (1H, large, NH), 4,1 (2H, dt J=7,8 Hz, CH₂OH), 2,46 (3H, CH3 tosyl), 2,08 (2H, m, CH₂CH₂OH), 1,81 (4H, m, CH₂CN), 1,63 (2H, CH₂), 1,55 (6H, CH2), 1,4-1,43 (9H, CH₃).

### Exemple 4 : Préparation N(3-(2-fluoro-ethyl) adamantane)-O-tertiobutyl carbamate (P2)

Le produit (P1) obtenu dans l'exemple 3 est utilisé comme produit de départ.

Dans un ballon tricol de 100 ml muni d'un condenseur et d'un thermomètre, on fait dissoudre, sous atmosphère d'argon anhydre, 225 mg de composé (P1) (0,5 mmol) est dilué dans 30 ml de dichlorométhane préalablement séché sur P₂O₅.

On baisse la température du milieu réactionnel à -42°C pour prévenir la forte exothermie de la réaction.

On introduit ensuite dans le ballon au goutte à goutte, 1 mmol (131 µl) de (diéthyl-amino)-sulfure trifluoride (désigné ensuite par « DAST »). On maintient le milieu à -42°C pendant 2 heures puis on laisse la température remonter à l'ambiante. Après 16 heures dans ces conditions, on abaisse la température à -78°C et on effectue lentement l'hydrolyse des réactifs avec une solution aqueuse de carbonate de potassium (12 ml d'eau pour 3,5g de K₂CO₃; 25 mmol). Après 2 heures on laisse la réaction revenir à température ambiante.

Après une heure dans ces conditions, on récupère la phase organique puis on lave 2 fois avec de l'eau à pH 7, on la sèche ensuite avec du sulfate de magnésium puis on concentre sous pression réduite pour obtenir une huile jaune pâle (66 mg, 45%).

On caractérise le produit par RMN comme étant le composé (P2):
¹H RMN (CDCl3, Bruker 300 MHz) : 4,50 (2H, dt J=7,8 Hz et J=43 Hz, CH₂F), 4,38 (1H, large, NH), 1,95-1,43 (25H, CH₃).

### Exemple 5 : Préparation du chlorhydrate de 1-amino-3-fluoroethyl-adamantane (II) et du 1-amino-3-fluoroéthyl-adamantane (I)

A partir du composé obtenu dans l'exemple 4, on réalise le mode opératoire suivant.

Dans un ballon de 50 ml on solubilise 100 mg de composé (P2) dans 3 ml de dichlorométhane. On baisse la température du milieu à 0°C puis on ajoute 10 ml d'éther éthylique chlorhydrique.

On ajoute après 8h à température ambiante, 10 ml d'une solution d'acide chlorhydrique 10⁻³ M. La phase aqueuse est récupérée et on recommence cette extraction 2 fois.

Les fractions sont rassemblées et concentrées sous vide. On obtient une huile (55 mg) qui est reprise dans 1 ml d'eau et on précipite le produit en ajoutant 1 ml d'éthanol. On récupère le précipité par filtration (50 mg, 75 %) qui est le composé (II) (caractérisé par spectrométrie de masse, M+= 197,1567 C₁₂H₂₀FN (calculé 197,158) ; m/z(Cl-) 196,15 (M-1), 181,1 (M-NH₂), 150,1 (M-C₂H₄F).

Le composé (II) est transformé en composé (I) selon le protocole suivant.

50 mg de composé (II) sont dilués dans 3ml d'eau. On amène le pH de la solution à 9 au moyen d'une solution 10% de soude. On extrait avec 6 ml de dichlorométhane, et on recommence 2 fois l'extraction. Les fractions sont rassemblées et séchées sur sulfate de magnésium. On évapore le solvant sous vide et on récupère le composé (I) sous forme d'une huile jaune pâle caractérisé par RMN :
¹H RMN (CDCl3, Brüker 400 MHz) : 4,54 (2H, dt J=12 Hz et J=48 Hz, CH₂F), 2,15 (2H, large, NH₂), 1,65-1,26 (16H, CH₃).

Pour les exemples suivants, on utilisera le terme de fluoroéthylnormémantine comme terme générique pour désigner le composé I ou son sel de chlorure II. La raison de ce choix est que le sel II est utilisé pour fabriquer les solutions injectables tandis que la molécule repérée dans les extraits de cellules est la molécule neutre hydrophobe.

### Exemple 6 : Propriétés de la ¹⁸F-fluoroéthyl normémantine le rendant apte pour être un marqueur des récepteurs NMDA l'imagerie médicale

### - Toxicité

L'objectif de cet essai est d'apprécier qualitativement et quantitativement les éventuels phénomènes toxiques et leur délai d'apparition après administration unique d'une dose prédéterminée de 0,125 µg/kg de poids corporel (ce qui correspondrait à plus de 10⁴ fois la dose administrée à l'homme). Les essais ont été menés sur 5 souris mâles et les résultats ont été comparés à ceux de 3 souris témoins qui ne reçoivent que 0.2ml de solution 0,9% de NaCl. Toutes les souris sont de l'espèce C57/Black 6J fournies par le laboratoire Charles River (France) et âgées environ de 8 semaines. Le poids moyen des souris en début des essais est d'environ 32g après un temps d'acclimatation de 2 semaines. Les animaux sont hébergés à raison de 3 par cage de 31 cm * 46 cm * 19 cm à une température de 25°C +/-2°C.

On a préparé une solution mère de (¹⁸F) Fluoroéthylnormémantine à 20 µg/l qui est répartie dans des flacons stériles de 15 ml. Le volume administré à chaque souris est défini à 0,125 µg/kg et est administré en une fois par intraveineuse à l'aide d'une seringue et d'une aiguille appropriées.

Les animaux sont observés régulièrement le jour de l'administration (après 30 mn, 1h, 2h, 3h et 4h), puis une fois par jour pendant au moins 14 jours. Toutes les observations ont donné : « rien à signaler ». Tous les animaux, à la fin des 14 jours ont été euthanasiés et autopsiés : aucune altération pathologique n'a été notée.

Ces essais permettent de conclure que la (¹⁸F)fluoroethylnormémantine n'est pas toxique aux doses testées. Comme ces doses sont très supérieures à celles envisagées pour le marquage des récepteurs NMDA, ce produit sera bien toléré pour cette application.

### Exemple 7 : Caractéristiques physico-chimiques de la ¹⁸F-fluoroéthylnormémantine

- Solubilité dans l'eau supérieure à 100 mM ce qui est favorable pour un injectable.
- Lipophilie : déterminée par log(P)= log ([I]_{n-octanol}/[I]ₑₐᵤ), en mettant le composé (I) dans un mélange 50/50 en volume d'eau tamponnée à un pH de 7,4 et d'octanol puis en séparant les phase et en dosant par HPLC le taux de composé (I) présent dans chaque phase. On trouve log(P)=3,1 (+/- 0,5) ce qui montre la lipophilie de I. Cette valeur est favorable à une pénétration dans le cerveau.

### Exemple 8 : Distribution dans l'organisme étude sur des souris

Les essais ont été menés sur des lots de 3 souris mâles et les résultats ont été comparés à ceux de 3 souris témoins qui ne reçoivent que 0.2ml de solution 0,9% de NaCl. Toutes les souris sont de l'espèce C57/Black 6J fournies par le laboratoire Charles River (France) et âgées environ de 8 semaines.

On a préparé une solution mère de (¹⁸F) Fluoroéthylnormémantine à 20 µg/l qui est répartie dans des flacons stériles de 15 ml. Le volume administré à chaque souris est défini à 0,125 µg/kg et est administré en une fois par intraveineuse à l'aide d'une seringue et d'une aiguille appropriées.

Au temps prescrit les souris sont sacrifiées et la concentration dans les organes est analysée après extraction d'un broyat de ces organes par des dosages HPLC couplée masse.

On obtient les résultats suivants en sacrifiant à 3 mn
Sacrifice à 3 mn :
   Cerveau : 12% de la dose injectée
   Reins : 18%
   Foie : 20%
Sacrifice à 30 et 60 mn:
   Ratio Cerveau/Sang (60mn) : 6,015

Par rapport à l'art antérieur on constate que la fluoroéthylnormémantine a une affinité forte pour le cerveau (la fluoromémantine a été mesurée à 3% au lieu de 12% ici) et que le ratio cerveau/sang est comparable.

Cette expérience permet de constater que la différence structurale entre la mémantine et le composé F-fluoroéthylnormémantine induit une différence de comportement de distribution dans les organes.

### Exemple 9 : Affinité relative de la ¹⁸F-fluoroéthylnormémantine pour les récepteurs NMDA.

Dans cet exemple on va comparer l'affinité de la fluoroéthylnormémantine pour les récepteurs NMDA au glutamate (Glu) et au produit Dizocilbine (dénommé « MK 801 ») qui sont des antagonistes connus des récepteurs NMDA (voir Neuroscience Letters, Vol. 80 (1), p. 111-114). Le MK801 se lie sur 2 sites différents dans le cerveau de rat (Brain Res. 378, p. 133).

Pour ce faire, un matériau contenant les récepteurs NMDA va être d'abord immergé dans une solution contenant le MK 801 (première expérience) ou le Glutamate (deuxième expérience) marqués puis ces matériaux sont incubés dans des solutions contenant la fluoroéthylnormémantine à des concentrations variables. On compare ensuite les émissions radioactives entre avant et après cette seconde incubation. Si les émissions ont diminué, cela signifie que la fluoroéthylnormémantine a plus d'affinité pour les récepteurs NMDA que les références et que des récepteurs NMDA sont inhibés par celle-ci.

On peut grâce à ces mesures établir une corrélation entre concentration de composé IE et inhibition des récepteurs NMDA. On en déduit ainsi la grandeur IC 50 qui est la demi-concentration inhibitrice maximale.

### Première expérience :

On utilise donc le composé MK 801 comme antagoniste (produit R0). On utilise comme produit à tester (produit RI) soit la mémantine soit le composé I. Le protocole expérimental est le suivant : on prépare un homogénat de membrane de cortex cérébral (140 µg de protéines) que l'on incube pendant 2h à 37°C avec 10 nM de [³H]phéncyclidine (MK801 tritié) en l'absence ou en présence du composé RI (dans un tampon contenant 5mM Hepes/Tris (Ph=7,4) et 0.1 mM d'acide éthylèneglycol tétraacétique). La comparaison se fait avec une incubation à 10 mM de MK801. Les échantillons sont filtrés rapidement sous vide à travers des filtres en fibre de verre et rincé avec 50mM de TrisHCl glacé. Les filtres sont séchés et leur radioactivité est comptée dans un compteur à scintillations. La concentration de composé RI varie et en comparant pour chaque concentration la différence d'émission du filtre témoin (MK801 seul) et du filtre du mélange MK801/R1 on obtient le ratio d'inhibition du composé RI pour chaque concentration. Le résultat de cette étude est résumé dans le tableau I suivant :

**Tableau I**

| R0 | R1 | [RI] Mol/l | ratio émission 1° essai (%) | ratio émission 1° essai (%) | Moyenne | IC 50 (Mol/l) |
|---|---|---|---|---|---|---|
| MK 801 | Mémantine | 1.10⁻⁹ | 97,8 | 95,5 | 96,9 | |
| | | 3.10⁻⁹ | 111,5 | 95 | 103,3 | |
| | | 1.10⁻⁸ | 105,5 | 97,3 | 101,4 | |
| | | 3.10⁻⁸ | 98,5 | 96,5 | 97,5 | |
| | | 1.10⁻⁷ | 86,2 | 91,4 | 88,8 | 1,1.10⁻⁶ |
| | | 3.10⁻⁷ | 77,1 | 69,2 | 73,1 | |
| | | 1.10⁻⁶ | 54,8 | 49,8 | 52,3 | |
| | | 3.10⁻⁶ | 27,9 | 30,1 | 29,0 | |
| | | 1.10⁻⁵ | 12,2 | 9,3 | 10,8 | |
| | Fluoroéthyl Normémantine | 1.10⁻⁹ | 107,3 | 98,9 | 103,1 | |
| | | 3.10⁻⁹ | 102,1 | 98,8 | 100,5 | |
| | | 1.10⁻⁸ | 98,4 | 107,7 | 103,1 | |
| | | 3.10⁻⁸ | 107,4 | 102,1 | 104,8 | |
| | | 1.10⁻⁷ | 92,5 | 98,6 | 95,5 | 6,1. 10⁻⁶ |
| | | 3.10⁻⁷ | 92,2 | 88,9 | 90,5 | |
| | | 1.10⁻⁶ | 79,4 | 91,8 | 85,6 | |
| | | 3.10⁻⁶ | 58,2 | 68,4 | 63,3 | |
| | | 1.10⁻⁵ | 42,9 | 41,5 | 42,2 | |

De cette mesure on voit que la fluoroéthylnormémantine est un inhibiteur des récepteurs NMDA puisqu'il déplace le MK 801. Son affinité est d'un ordre de grandeur comparable à celui de la mémantine (10⁻⁶ M). Lorsque la fluoroéthylnormémantine sera fonctionnalisée par un atome de fluor 18, il pourra donc, en se fixant sur les récepteurs NMDA libres permettre de repérer ceux-ci en utilisant un scanner à émission de positrons. On peut donc imaginer que dans le cas où l'on souhaiterait étudier l'effet d'un nouveau médicament sur ces récepteurs, on puisse cartographier les récepteurs qui restent libres en présence de de nouveau médicament.

### Deuxième expérience :

Le protocole expérimental est à peu près le même que dans la première expérience : on prépare un homogénat de membrane de cortex cérébral (140 µg de protéines) que l'on incube pendant 1h à 4°C avec 5 nM de glutamate tritié (Glu tritié) en l'absence ou en présence de la fluoroéthylnormémantine (dans un tampon contenant 5mM TrisHCl (Ph=7,7) et 10 mM d'acide éthylèneglycol tétraacétique). La comparaison se fait avec une incubation à 100 mM de L-Glu tritié. A la suite de l'incubation, les échantillons sont filtrés rapidement sous vide à travers des filtres en fibre de verre et rincé avec 50mM de TrisHCl glacé. Les filtres sont séchés et leur radioactivité est comptée dans un compteur à scintillations. La concentration de composé RI varie et en comparant pour chaque concentration la différence d'émission du filtre témoin (MK801 seul) et du filtre du mélange MK801/R1 on obtient le ratio d'inhibition du composé RI pour chaque concentration. Le résultat de cette étude est résumé dans le tableau II suivant :

**Tableau II**

| R0 | R1 | [R1] (M) | ratio émission 1° essai (%) | ratio émission 1° essai (%) | Moyenne |
|---|---|---|---|---|---|
| Glu | Fluoro -éthyl normémantine | 1.10⁻⁹ | 106,4 | 88,8 | 97,6 |
| | | 3.10⁻⁹ | 101,9 | 101,6 | 101,8 |
| | | 1.10⁻⁸ | 92,3 | 105,7 | 99,0 |
| | | 3.10⁻⁸ | 108,0 | 91,0 | 99,5 |
| | | 1.10⁻⁷ | 108,4 | 111,0 | 109,7 |
| | | 3.10⁻⁷ | 94,2 | 109,0 | 101,6 |
| | | 1.10⁻⁶ | 105,3 | 100,2 | 102,8 |
| | | 3.10⁻⁶ | 104,3 | 92,5 | 98,4 |
| | | 1.10⁻⁵ | 95,4 | 108,6 | 102,0 |

Ces résultats montrent que le Glu est un meilleur inhibiteur que la fluoroéthyl normémantine . On ne peut donc mesurer de IC50 dans ce cas.

Ces deux expériences montrent que la fluoroéthylnormémantine a une affinité pour les récepteurs NMDA qui permettrait d'utiliser son dérivé marqué au fluor 18 pour faire de l'imagerie par scanner à émission de positrons. Sa capacité à déplacer des inhibiteurs comme le MK801 sans être capable de déplacer le Glu permet de cibler l'utilisation de ce composé pour analyser des mécanismes d'action de médicaments sur des maladies psychiatriques telles que la maladie d'Alzheimer, la schizophrénie, la maladie de Parkinson ou d'autres par PET Scan par exemple.

## Revendications

1. Composé chimique de formule (I) : 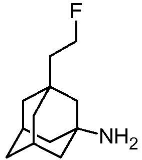 ou un de ses sels de formule (II) : 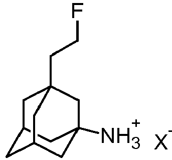 formule dans laquelle, X⁻ désigne un contre anion issu du milieu biologique ou choisi parmi les ions chlorure, bromure, iodure, acétate, méthane sulfonate, benzène sulfonate, camphosulphonate, tartrate, dibenzoate, ascorbate, fumarate, citrate, phosphate, salicylate, oxalate, bromohydrate, tosylate.

2. Composés selon la revendication 1 dans lesquels l'atome de fluor est un atome de fluor 18 (¹⁸F)

3. Solution aqueuse des composés de la revendication 1 utilisable pour être injectée en intraveineuse.

4. Composé de formule P2 en tant que précurseur des composés selon la revendication 1 ou 2 :

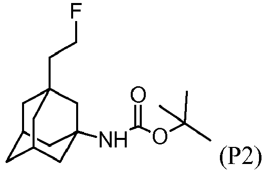

5. Composé de formule P1 en tant que précurseur du composé défini dans la revendication 4

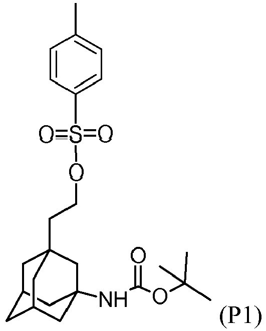

6. Utilisation des solutions obtenues selon la revendication 3 en injection à un mammifère pour réaliser un scanner à émission de positron, par repérage des atomes de ¹⁸F pour étudier le comportement des récepteurs NMDA lors d'étude sur des maladies neurologiques.

7. Composés des revendications 1, 2, 4 ou 5 pour une utilisation dans des compositions médicamenteuses destinées au traitement des maladies neurologiques.

8. Composé (I) ou (II) pour son utilisation en tant que médicament.

## Patentansprüche

1. Chemische Verbindung der Formel (I): 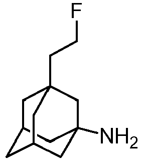 oder eines ihrer Salze der Formel (II): 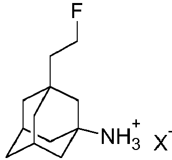 Formel, in welcher X- ein Gegenanion bezeichnet, hervorgegangen aus dem biologischen Milieu oder ausgewählt aus den Chlorid-, Bromid-, Jodid-, Acitat-, Methansulfonat-, Benzolsulfonat-, Camphosulphonat-, Tartrat-, Dibenzoat-, Ascorbat-, Fumarat-, Citrat-, Phosphat-, Salicylat-, Oxalat-, Bromohydrat-, Tosylationen.

2. Verbindungen nach Anspruch 1, wobei das Fluoratom ein Fluoratom 18 (¹⁸F) ist.

3. Wässrige Lösung der Verbindungen von Anspruch 1, als intravenöse Injektion verwendbar.

4. Verbindung der Formel P2 als Vorläufer der Verbindungen nach Anspruch 1 oder 2:

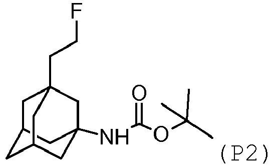

5. Verbindung der Formel P1 als Vorläufer der in Anspruch 4 definierten Verbindung

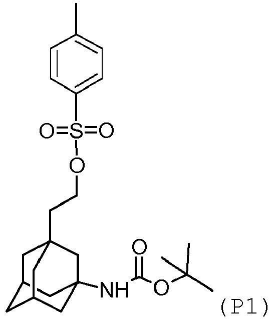

6. Verwendung der Lösungen nach Anspruch 3 als Injektion in einen Säuger zwecks Durchführung einer Positronenemissionstomographie mittels Feststellung von ¹⁸F-Atomen zur Untersuchung des Verhaltens der NMDA-Rezeptoren bei Untersuchung von neurologischen Erkrankungen.

7. Verbindungen der Ansprüche 1, 2, 4 oder 5 für eine Verwendung in medikamentösen Zusammensetzungen, die zur Behandlung von neurologischen Erkrankungen bestimmt sind.

8. Verbindung (I) oder (II) für ihre Verwendung als Arzneimittel.

## Claims

1. Chemical compound of formula (I): 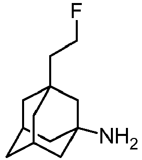 or a salt thereof of formula (II): 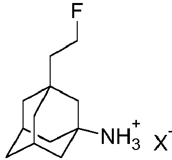 formula wherein X⁻ indicates a counteranion from the biological environment or selected from the ions chloride, bromide, iodide, acetate, methane sulphonate, benzene sulphonate, camphosulphonate, tartrate, dibenzoate, ascorbate, fumarate, citrate, phosphate, salicylate, oxalate, bromohydrate, tosylate.

2. Compound according to claim 1, wherein the fluorine atom is a fluorine-18 (¹⁸F) atom.

3. Aqueous solution of the compounds of claim 1 which can be injected intravenously.

4. Compound of formula P2 as a precursor of the compounds according to claim 1 or 2:

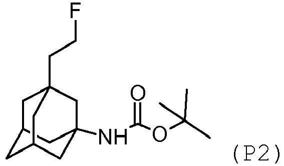

5. Compound of formula P1 as a precursor of the compound defined in claim 4:

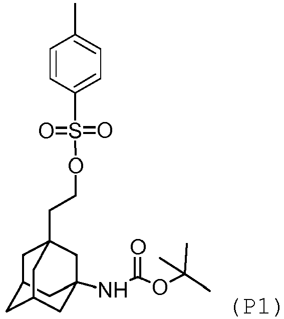

6. Use of the solutions obtained according to claim 3 for injection in a mammal in order to carry out a positron emission scanner to locate the ¹⁸F atoms in order to study the behavior of NMDA receptors during the study of neurological diseases.

7. Compounds of claims 1, 2, 4 or 5 for use in drug compositions intended for the treatment of neurological diseases.

8. Compound (I) or (II) for use as a drug.
